(19) Europäisches Patentamt
European Patent Office
Office européen des brevets

(11) Publication number: **0 284 800**
**A1**

(12) **EUROPEAN PATENT APPLICATION**

(21) Application number: **88103130.6**

(51) Int. Cl.[4]: **C07D 211/58** , C08K 5/34

(22) Date of filing: **02.03.88**

The title of the invention has been amended (Guidelines for Examination in the EPO, A-III, 7.3).

(30) Priority: **05.03.87 US 22183**

(43) Date of publication of application:
**05.10.88 Bulletin 88/40**

(84) Designated Contracting States:
**AT BE CH DE FR GB IT LI LU NL SE**

(71) Applicant: **The B.F. Goodrich Company**
**3925 Embassy Parkway**
**Akron Ohio 44313(US)**

(72) Inventor: **Lai, John Ta-Yuan**
**3465 Ridge Park Blvd.**
**Broadview Heights Ohio 44147(US)**
Inventor: **Son, Pyong Nae**
**2106 Ayers Avenue**
**Akron Ohio 44313(US)**

(74) Representative: **von Kreisler, Alek et al**
**Patentanwälte Von Kreisler-Schönwald-Fues-Keller Selting-Werner Deichmannhaus am Hauptbahnhof**
**D-5000 Köln 1(DE)**

# (54) N-(substituted piperidinyl)-alpha-(3,5-dialkyl-4-hydroxyphenyl)-alpha', alpha"-dialkyl acetamides.

(57) N-(2,2,6,6-tetraalkyl-4-piperidinyl)-α-(3,5-di-alkyl-4-hydroxyphenyl)-α',α"-dialkylacetamides are prepared by a new process are novel stabilizers for organic materials subject to attack and degradation, and form useful combinations for this purpose with other stabilizers against attack of polymer materials by heat, oxygen and light.

EP 0 284 800 A1

# N-(SUBSTITUTED CYCLIC ALKYLENEIMINE)-α -(3,5-DI-ALKYL-4-HYDROXYPHENYL)-α′,α″-DIALKYL ACETAMIDES

This is a continuation-in-part of Application Serial Number 713,715, filed March 20, 1985, a division of Number 549,036, filed November 7, 1983.

## SUMMARY OF THE INVENTION

Novel N-(substituted cyclic alkyleneimine)-α-(3,5-di-alkyl-4-hydroxyphenyl)-α′,α″-dialkyl acetamides prepared by a new process are novel stabilizers for organic materials subject to attack and degradation by heat, oxygen and light, and form useful combinations for this purpose with other hindered phenol stabilizers.

## DETAILED DESCRIPTION

The novel N-(substituted cyclic alkyleneimine)-α-(3,5-di-alkyl-4-hydroxyphenyl)-α′, α″-dialkyl acetamides of this invention have the general formula

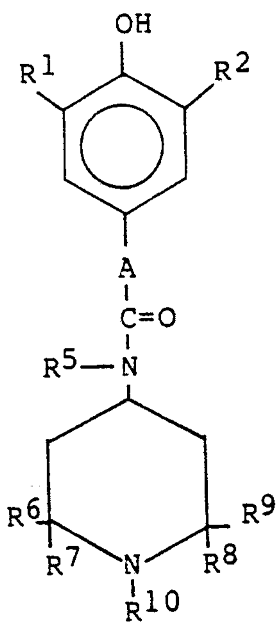

wherein $R^1$ and $R^2$ are hydrogen or alkyl and cycloalkyl groups containing 1 to 12 and 3 to 12 carbon atoms re spectively, alkylcycloalkyl and aryl or alkaryl groups wherein the alkyl groups contain 1 to 8 carbon atoms, and the aryl groups are normally phenyl or naphthyl; $R^5$, $R^6$, $R^7$, $R^8$ and $R^9$ are hydrogen, alkyl and cycloalkyl groups containing 1 to 12 and 3 to 12 carbon atoms respectively, alkylcycloalkyl and aryl or alkaryl groups wherein the alkyl groups contain 1 to 8 carbon atoms, and the aryl groups are normally phenyl or naphthyl; A is (1) an alkylidene group of the formula $R^3-C-R^4$ wherein $R^3$ and $R^4$ are alkyl groups containing 1 to 18 carbon atoms, or (2) aryl or alkaryl groups wherein the alkyl groups contain 1 to 8 carbon atoms, and the aryl groups, normally are phenyl or naphthyl, cycloalkane groups containing 5 to 12 carbon atoms, including for example those cycloalkane groups derived from cyclohexanone, cycloheptanone, and the like; alkyl aryl ketones wherein the alkyl groups contain 1 to 8 carbon atoms and the aryl preferably is phenyl or naphthyl; $R^{10}$ is hydrogen or alkyl radicals containing 1 to 18 carbon atoms, or acyl group containing 1 to 18 carbon atoms, O or OH. Preferably, $R^1$ is an alkyl group containing 1 to 8 carbon atoms, and $R^2$ is an alkyl group containing 1 to 5 carbon atoms, and more preferably at least one of $R^1$ or $R^2$ is a t-alkyl group including t-butyl and t-amyl; $R^3$ and $R^4$ are alkyl groups containing 1 to 8 carbon atoms, and more preferably 1 to 4 carbon atoms; $R^5$ is an alkyl group containing 1 to 6 carbon atoms; $R^6$ and $R^7$ are methyl; $R^8$ is an alkyl group containing 1 to 3 carbon atoms; $R^9$ is an alkyl group containing 1 to

4 carbon atoms; and $R^{10}$ is hydrogen.

These N-(substituted cyclic alkyleneimines)-α-(3,5-di-alkyl-4-hydroxyphenyl)-α′,α″-dialkyl acetamides, more specifically the N-(2,2,6,6-tetraalkyl-4-pipereidinyl)-α-(3,5-di-alkyl-4-hydroxyphenyl)-α′,α″-di-alkylacetamides, are prepared by a new process by reacting a 2,6-di-alkylphenol with an aliphatic, cycloaliphatic or alkaryl ketone, a haloform and a piperidine compound in the presence of alkali metal hydroxide. An organic solvent may be used, or large amounts of the ketone reactant may be employed. The amide is isolated in excellent yields by crystallizing the filtered reaction product.

Typical 2,6-dialkyl phenols used have the formula

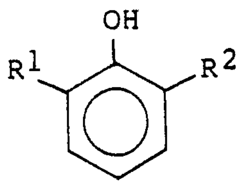

wherein $R^1$ and $R^2$ have the meanings set forth above, including 2-methyl-6-t-butylphenol, 2-ethyl-6-t-butylphenol, 2-propyl-6-t-butylphenol, 2-isopropyl-6-t-butylphenol, 2-n-butyl-6-t-butylphenol, 2,6-di-t-butylphenol, 2-n-amyl-6-t-butylphenol, 2-isoamyl-6-t-butylphenol, 2-hexyl-6-t-butylphenol, 2-heptyl-6-t-butylphenol, 2-isooctyl-6-t-butylphenol, 2-isopropyl-6-methylphenol, 2-n-butyl-6-isopropylphenol, 2-isoamyl-6-ethylphenol, 2-isoamyl-6-methylphenol, 2-isooctyl-6-methylphenol, 2-isooctyl-6-ethylphenol, 2-isooctyl-6-n-propylphenol, 2-isooctyl-6-n-hexylphenol, and the like.

The ketones used include dialkyl ketones, cycloalkanones, alkylcycloalkanones, and alkyl aryl ketones. Typical ketones that may be used in the novel process to make the new 3,5-dialkyl-4-hydroxyphenyl-substituted acetic acid derivatives of the invention are alkyl ketones of the formula

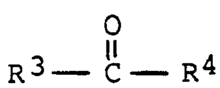

wherein $R^3$ and $R^4$ are alkyl radicals containing 1 to 18 carbon atoms, preferably 1 to 8 carbon atoms, including for example acetone, methyl ethyl ketone, methyl n-propyl ketone, diethyl ketone, 2-hexanone, 3-hexanone, di-n-propyl ketone, 2-octanone, methyl isopropyl ketone, and the like. Also useful are the cycloalkane ketones containing 5 to 12 carbon atoms in the cyclic ring such as cyclopentanone, cyclohexanone, cycloheptanone, cyclooctanone, cyclodecanone, methylcyclopentanone, methylcyclohexanone, dicyclohexyl ketone, cyclododecanone; also alkyl aryl ketones when the alkyl group contains 1 to 4 carbon atoms such as acetophenone, o-methoxyacetophenone, p-chloroacetophenone, and the like. The amounts used are from at least about 1 mole of ketone per mole of 2,6-dialkylphenol, up to amounts sufficient for the ketones to be the solvent of the reaction, 10 moles or more. No more than about two moles is preferred when the ketone is a reactant only. Use of less than 1 mole reduces the product yield, and more than about 2 moles is unnecessary unless the ketone is the solvent.

The haloform, such as chloroform, is also used in a molar ratio of at least about one mole per mole of 2,6-dialkylphenol used, but preferably a slight molar excess is used up to about a 50 percent molar excess, i.e., 1.5 mole per mole of 2,6-dialkylphenol. While larger amounts may be used, there is no advantage realized, and lesser amounts may decrease the ultimate yield of the desired product. Bromoform may be substituted for the chloroform and excellent results will be obtained.

The alkali metal hydroxide is used in powder form, or in solution, and includes sodium hydroxide and potassium hydroxide. Preferably, a molar excess of the alkali metal hydroxide is used in relation to the amount of 2,6-dialkylphenol present. Normally from about 4 moles of alkali metal hydroxide per mole of 2,6-dialkylphenol up to 10 or more moles may be used, but the amount used preferably is about 4 to about 8 moles of hydroxide per mole of 2,6-di-alkylphenol. Use of less than about 4 moles will reduce the yield of desired product.

The N-substituted cyclic alkyleneimines used in the process of this invention to make the novel acetamides, the piperidines have the general formula .

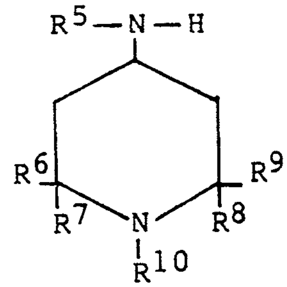

wherein $R^5$, $R^6$, $R^7$, $R^8$ and $R^9$ are hydrogen, alkyl, cycloalkyl, aryl or alkaryl radicals containing 1 to 18 carbon atoms, about at least 3 carbon atoms in the cycloalkyls and at least 6 carbon atoms in the aryls. More preferably the alkyl groups contain 1 to 8 carbon atoms, $R^{10}$ is hydrogen, alkyl, acyl, or alkaryl as defined above, oxygen or hydroxyl. Typical compounds are substituted piperidine derivatives such as 4-amino-2,2-6,6-tetramethyl piperidine, 4-methylamino-2,2,6,6-tetramethyl piperidine, 4-ethylamino-2,2,6,6-tetramethyl piperidine, 4-butylamino2,2,6,6-tetramethyl piperidine, N,N'-bis-(2,2,6,6-tetramethyl-4piperidyl)-1,6-hexanediamine, and the like.

The amount of substituted piperidine used in the reaction is based on one mole of the 2,6-dialkylphenol. While the amount may vary from less than a stoichiometric amount, resulting in lower product yield, to larger excesses that are not required and have to be separated from the reaction mixture, normally about 0.5 to 2.5 moles to about 1 mole is used.

The solvent used may be any polar organic solvent, including an excess of the ketone used in place of an added solvent. Typical solvents that may be used include methylene chloride, tetrahydrofuran, diethyl ether, dibutyl ether, dimethyl sulfoxide, 1,4-dioxane, carbon tetrachloride, toluene, xylene, and the like. The amounts of solvent used will vary from about 5 moles to 100 moles per mole of 2,6-dialkylphenol used. As has been stated, the solvent may be eliminated if an excess of the reactant ketone is used. In this case, the amount of ketone used, based on the moles of 2,6-dialkylphenol used may be from about 5 to about 20, preferably about 7.5 to 15 moles per mole of 2,6-dialkylphenol.

While the reactants may be added in any order, it is preferred that the alkali metal hydroxide be added last, over a period of time to control the exothermic reaction and preferably maintain the reaction below 30°C, preferably below about 10°C. The reaction temperature may be varied from about 0°C to about 30°C, but preferably is conducted from about 5°C to 15°C. The reaction time normally will vary from about 5 to about 15 hours.

The amides are readily isolated from the reaction mixture by concentrating the reaction mixture, followed by stirring the residue in water, where the product was isolated in solid form. This product may be recrystallized if desired.

The practice of the invention is demonstrated in the following Examples. The structures of the amides of the following Examples were confirmed by infrared and nuclear magnetic resonance spectra. Molecular weights were determined and confirmed by field desorption mass spectra (FD/MS). Elemental analysis of carbon and hydrogen was done in most compounds and the amounts found were consistent with the formulas of the materials.

EXAMPLE 1

N-methyl-N'-(2,2,6,6-tetramethyl-4-piperidinyl)-2-(3,5-di-t-butyl-4-hydroxyphenyl)-2-methyl propionamide

0.1 Mole of 2,6-di-t-butylphenol, 1.0 mole of acetone, 0.15 mole of chloroform and 0.1 mole of 4-methylamino-2,2,6,6-tetramethylpiperidine were added to a reactor and mixed by stirring while being cooled to 5°C by a circulating cold bath. 0.5 mole of powdered sodium hydroxide was slowly added in small portions over a period of 1 hour. The reaction mixture was stirred at 10°C overnight. The reaction mix from the reactor was filtered. The solid residue was washed with methylene chloride and the wash added to the filtrate. The filtrate was washed with 50 ml of 5% sodium carbonate, and was then dried over sodium sulfate. The filtrate was evaporated to dryness and the dried product was washed with hexane. The resulting amide was a white solid that had a melting point of 168-173°C and a molecular weight of 444. By elemental analysis it was determined that the amide contained 75.38% carbon (75.67% calculated), 10.76% hydrogen (calculated 10.88%) and 6.20% nitrogen (calculated 6.30%).

EXAMPLE 2

Bis-{N-(2,2,6,6-tetramethyl-4-piperidinyl)-N'-[2-(3,5-di-t-butyl-4-hydroxyphenyl)-2-methyl propaneoyl]}, 1,6-hexanediamine

0.4 mole of 2,6-di-t-butylphenol, 4.0 moles of acetone, 0.4 mole of chloroform, and 0.1 mole of 4-hexamino-2,2,6,6-tetramethyl piperidine were added to a reactor and mixed by stirring while being cooled by a circulating cold bath. 1.4 mole of powdered sodium hy droxide was slowly added in small portions over a period of 1 hour. The reaction mixture was stirred at 10°C overnight. The reaction mix from the reactor was filtered. The solid residue was washed with acetone then stirred in 1.5 liters of water. The solid was collected and was washed with water. It can be further purified by recrystallization from toluene. The resulting amide was a white solid that had a melting point of 255-260°C and a molecular weight of 983.5. By elemental analysis it was determined that the amide contained 76.40% carbon (76.38% calculated), 10.71% hydrogen (calculated 10.90%) and 5.79% nitrogen (calculated 5.84%).

EXAMPLE 3

N-methyl-N'-(2,2,5,5-tetramethyl-4-piperidinyl)-(3,5-di-t-butyl-4-hydroxyphenyl)-cyclohexanecarboxamide

0.15 mole of 2,6-di-t-butylphenol, 1.2 mole of cyclohexanone, 0.15 mole of chloroform and 0.1 mole of 4-methylamino-2,2,6,6-tetramethylpiperidine were stirred in a reactor while being cooled to about 5°C. 0.5 mole of sodium hydroxide beads were added in portions to keep the temperature below 10°C. The reaction mixture was stirred for 10 hours. 250 ml of water was added and the mixture was stirred for 20 minutes, filtered and the solid product was rinsed with water. It can be further purified by recrylstallization from 95% ethanol. More product can be recovered from the organ ic phase in the filtrate by distillation of volatile reagents and washed the residue with heptane. The purified product was a white solid with a melting point of 153°C.

EXAMPLE 4

Bis-{N-(2,2,6,6-tetramethyl-4-piperidinyl)-N'-[2-(3,5-di-t-butyl-4-hydroxyphenyl)-2-methylbutanoyl]},-1,6-hexanediamine

0.4 mole of 2,6-di-t-butylphenol, 4.0 moles of 2-butanone, 0.4 mole of chloroform and 0.1 mole of bis-[N-(2,2,6,6-tetramethyl-4-piperidinyl]-1,6-hexanediamine were stirred and cooled at 5°C. 1.2 moles of sodium hydroxide beads was added in 2 hours. After being stirred at 10°C overnight, the reaction mixture was concentrated and the residue was stirred in 2 liters of water. The remaining solid was collected and washed with water and heptane. The white solid product melts at 227-231°C. This can be further purified by recrystallization from 90% ethanol. H'NMR and IR confirmed the structure.

EXAMPLE 5

Bis-{N-(2,2,6,6-tetramethyl-4-piperidinyl)-N'-[2-(3,5-di-t-butyl-4-hydroxyphenyl)-2-methylpentanoyl]}-1,6-hexanediamine

Following Example 4, 2-butanone was replaced by 2-pentanone. The resulting product was a white pow der with melting point of 159-163°C.

EXAMPLE 6

Bis-{N-2,2,6,6-tetramethyl-4-piperidinyl)-N'-[2-(3,5-di-t-butyl-4-hydroxyphenyl)-2-methylbutanoyl]}-2-methyl-1,5-pentanediamine

Following Example 4, bis-[N-(2,2,6,6-tetramethyl-4-piperidinyl)]-1,6-hexanediamine was replaced by bis-[N-(2,2,6,6-tetramethyl-4-piperidinyl)]-2-methyl-1,5-pentanediamine. The product was a white solid that melted at 144-148°C.

EXAMPLE 7

N-butyl-N'-(2,2,5,5-tetramethyl-4-piperidinyl)-2-(3,5-di-t-butyl-4-hydroxyphenyl)-2-methylbutanamide

Following Example 4, bis-[N-(2,2,6,6-tetramethyl-4-piperidinyl)]-1,6-hexanediamine was replaced by 4-N-butylamino-2,2,6,6-tetramethyl piperidine. The product was a white solid that melted at 149-158°C.

EXAMPLE 8

N-dodecyl-N'-(2,2,5,5-tetramethyl)-4-piperidinyl)-α-(3,5-di-t-butyl-4-hydroxyphenyl)-cyclohexanecarboxamide

0.03 mole of 2,6-di-t-butylphenol, 0.3 mole of cyclohexanone, 0.045 mole of chloroform and 0.06 mole of 4-N-dodecylamino-2,2,6,6-tetramethylpiperidine were mixed and cooled in a water bath. 0.15 mole of sodium hydroxide powders were added in 0.5 hour. The reaction was stirred at ambient temperature under nitrogen overnight. 50 ml each of toluene and water were then added and the layers were separated. The aqueous layer was extracted with 50 ml of toluene and the combined organic layers were washed with 50 ml saturated sodium chloride solution, dried over sodium sulfate and concentrated to dryness. Cyclohexanone and excess 4-N-dodecylamino-2,2,6,6-tetramethylpiperidine were removed by distillation. The residue was stirred in hexanes and product precipitated as a white powder. The melting point was 119-122°C.

EXAMPLE 9

N-ethyl-N'-(2,2,6,6-tetramethyl-4-piperidinyl)-2-(3,5-di-t-butyl-4-hydroxyphenyl)-2-methyloctanamide

Following Example 8, cyclohexanone was rereplaced with 2-octanone and 4-N-dodecylamino-2,2,6,6-tetramethylpiperidine was replaced with 4-N-ethylamino-2,2,6,6-tetramethylpiperidine. The product melted at 125-128°C.

EXAMPLE 10

N-ethyl-N'-(2,2,6,6-tetramethyl-4-piperidinyl)-2-(3,5-di-t-butyl-4-hydroxyphenyl)-2-methylbutanamide

Following Example 4, bis-[N-(2,2,6,6-tetramethyl-4-piperidinyl)]-1,6-hexanediamine was replaced by N-4-methylamine-2,2,6,6-tetramethylpiperidine. The product was a white powder which melted at 183-186°C.

EXAMPLE 11

N-ethyl-N'-(1,2,2,6,6-pentamethyl-4-piperidinyl)-2-(3,5-butyl-4-hydroxyphenyl)-2-methylbutanamide

Product from Example 10 (0.02 mole) was mixed with 15 ml 97% formic acid and 0.04 mole of paraformaldehyde. The mixture was heated to 80°C for 5 hours, then was concentrated to dryness. 50 ml of water was added followed by dropwise addition of 50% NaOH until the mixture became basic. The solid was collected and washed with water. After drying it was further recrystallized from toluene. The melting point was 212-216°C.

EXAMPLE 12

N-ethyl-N'-(1-acetyl-2,2,6,6-tetramethyl-4-piperidinyl)-2-(3,5-di-t-butyl-4-hydroxyphenyl)-2-methylbutanamide

Product from Example 10 (0.03 mole) and acetic anhydride (0.45 mole) were refluxed for 10 hours. The mixture was then concentrated and the residue was dissolved in 200 ml methylene chloride. The solution was washed with 100 ml 10% NaOH, and 50 ml of saturated NaCl solution, dried over $Na_2SO_4$ and concentrated. The solid was recrystallized from 60% ethanol to yield white powders. The melting point was 143-145°C.

To demonstrate the stabilizing activity of the amides of this invention, test samples of the amides in polypropylene were prepared by mixing the stabilizer compounds with polypropylene in a Brabender Plasticorder fitted with a Cam-Head (mixing chamber). The polypropylene is first masticated for 1 1/2 minutes at 190°C. Then the stabilizer is added, followed by 3 minutes additional mixing. The mass is removed and pressed into 20 mil thick sheets. From these sheets are cut 1" × 1" plaques for oven aging. Type C (3" × 1/8") tensil bars are cut for UV stability tests.

Thermal/oxidative stability (oven aging) testing consisted of aging the samples in triplicate in an air-circulating oven at 125°C. The time to catastrophic crumbling (failure) of the plaque was measured and reported as days to failure. Each sample contained 0.1 weight part of the named amide per 100 weight parts of polypropylene (pHr). The following results, in days to failure, were obtained:

| | 125°C |
|---|---|
| Control | 2 |
| N-methyl-N'-(2,2,6,6-tetramethyl-4-piperidinyl)-2-(3,5-di-t-butyl-4-hydroxyphenyl)-2-methylpropionamide (Example 1) | 7 |
| Bis-{N-(2,2,6,6-tetramethyl-4-piperidinyl)-N'-[2-(3,5-di-t-butyl-4-hydroxyphenyl)-2-methylpropanol]}-1,6-hexanediamine (Example 2) | 37 |

Samples containing 0.1 weight part of the amides listed below were tested for ultraviolet light stability, i.e., resistance to degradation by UV radiation. The samples were tested in an Atlas Xenon Weatherometer, Model No. 65-WR, equipped with a 6500 watt Xenon burner tube in accordance with ASTM #D2565-79-A. The black panel temperature was 60°C. The samples were subjected to an 18-minute water cycle every 2 hours. The time in hours to a 50% loss in tensile strength was determined. For comparison purposes a samples with no amide was tested. The following results, in hours, were obtained:

| | Hours |
|---|---|
| Blank (Control) | 220 |
| N-methyl-N'-(2,2,6,6-tetramethyl-4-piperidinyl)-2-(3,5-di-t-butyl-4-hydroxyphenyl)-2-methylpropionamide (Example 1) | 2450 |
| Bis-{N-(2,2,6,6-tetramethyl-4-piperidinyl)-N'-[2-(3,5-di-t-butyl-4-hydroxyphenyl)-2-methylpropanol]}-1,6-hexanediamine (Example 2) | 1980 |

In another UV test on polypropylene fibers, each sample was prepared to contain 0.1 phr each of calcium stearate, tris-[3,5-di-t-butyl-4-hydroxyphenyl]isocyanurate and the hindered piperidine light stabilizer.

| | Hours |
|---|---|
| Control | 340 |
| N-methyl-N'-(2,2,5,5-tetramethyl-4-piperidinyl)--(3,5-di-t-butyl-4-hydroxyphenyl)-cyclohexane-carboxamide (Example 3) | 1060 |
| Bis-{N-(2,2,6,6-tetramethyl-4-piperidinyl)-N'-[2-(3,5-di-t-butyl-4-hydroxyphenyl)-2-propanoyl]}-1,6-hexanediamine (Example 2) | 900 |
| Bis-{N-(2,2,6,6-tetramethyl-4-piperidinyl)-N'-[2-(3,5-di-t-butyl-4-hydroxyphenyl)-2-methylbutanoyl]}-1,6-hexanediamine (Example 4) | 940 |

The N-(2,2,6,6-tetraalkyl-4-piperidinyl)-α-(3,5-di-alkyl-4-hydroxyphenyl)-α',α"-dialkylacetamides are effective in protecting organic materials subject to degradation by heat, light, ozone, etc. Organic materials which can be stabilized in accordance with the present invention include both natural and synthetic polymers. For example, the stabilizers are useful for the stabilization of cellulosic materials; natural rubber, halogenated rubber, conjugated diene polymers, as for instance, polybutadiene, copolymers of butadiene with styrene, acrylonitrile, acrylic acid, alkyl acrylates or methacrylates, methyl vinyl ketone, vinyl pyridine, and the like; polyisoprene, polychloroprene, and the like; vinyl polymers such as polyvinyl chloride, polyvinylidene chloride, copolymers of vinyl chloride with vinylidene chloride, copolymers of vinyl halides with butadine, styrene, vinyl esters, α,β -unsaturated ketones and aldehydes, alkylacrylates, and the like; polymers of vinyl acetate; homopolymers and copolymers of acrylic monomers such as methyl acrylate, methyl methacrylate, ethyl acrylate, 3-ethylhexyl acrylate, acrylamide, methacrylamide, N-methylol-acrylamide, acrylonitrile, methacrylonitrile, and the like; epihalohydrin polymers; polyether-or polyol-derived polyurethanes; acetal homo-polymers and copolymers; polycarbonates; polyesters such as those derived from maleic, fumaric, itaconic, or terephthalic anhydrides, or the like, for example, polyethylene terephthalate; polyamides such as those derived from the reaction of hexamethylenediamine with adipic or sebacic acid; melamine; epoxy resins such as those obtained from the condensation of epihalohydrin with bisphenols; ring opened olefin polymers and the like. They are also useful for stabilizing coatings of these polymers, for example, polyurethanes, polycarbonates, acrylates, and melamines. Polymer blends, that is, physical admixture of two or more polymers may also be stabilized in accordance with the present invention.

In addition to polymeric materials, the present compounds may stabilize a wide variety of other organic materials. Such compounds include: colorants such as azo type pigments; waxes, synthetic and petroleum-derived lubricating oils and greases; animal oils such as, for example, fat, tallow, lard, codliver oil, sperm oil; vegetable oils such as castor, linseed, peanut, palm, cotton seed, and the like; fuel oil, diesel oil, gasoline and other petroleum products, and the like.

The N-(substituted cyclic alkyleneimine)-α-(3,5-dialkyl-4-hydroxyphenyl)α',α"-dialkyl acetamides as defined herein provide exceptional heat stability and resistance to ultraviolet degradation of polyolefin polymers. They are especially useful for the stabilization of α-monoolefin homopolymers and copolymers, wherein the α-monoolefin contains 2 to about 8 carbon atoms. High and low-density polyethylene, isotactic and atactic polypropylene, polyisobutylene, and poly(4-methyl-1-pentene) have excellent resistance to heat and oxygen when stabilized with the combinations of the present invention. Also, ethylene-propylene copolymers and ethylene-propylene terpolymers, generally containing less than about 10 percent by weight of one or more monomers containing multiple unsaturation provided, phenols; ring opened olefin polymers and the like. Polymer blends, that is, physical admixture of two or more polymers may also be stabilized in accordance with the present invention.

The (3,5-dialkylene-4-hydroxyphenyl)-substituted amides may be used with other stabilizers including hindered or partially hindered phenols, hindered amine light stabilizers, phosphites, o-hydroxybenzophenones, and the like. Typical hindered phenols are the hydroxyphenylalkyleneyl isocyanurates such as the sym metrical tris(3,5-di-t-alkyl-4-hydroxybenzyl) isocyanurates; tetrakis[methylene 3-(3',5'-dialkyl-4'-hydroxyphenyl)propanoate] methanes wherein the alkyl groups contain 1 to 8 carbon atoms, such as

tetrakis[methylene-3-(3′,5′-dit-butyl-4′-hydroxyphenyl)propanoate]methane; alkyl (3-3′,5′-di-t-butyl-4′-hydroxyphenyl)propionates wherein the alkyl groups contain 1 to 18 carbon atoms, such as octadecyl 3-(3′,5′-di-t-butyl-4-hydroxyphenyl)propionate; 1,3,5-trimethyl-2,4,6-tris[3,5-dialkyl-4-hydroxybenzyl)benzene; 1,3,5-tris-(3,5-di-t-butyl-4-hydroxyhydrocinnamoylethyl)-s-triazine-2,4,6-(1H,3H,5H)-trione; 2,2′-alkylidene bis (4,6-dialkylphenol)-s wherein the alkyl group contains 1 to 8 carbon atoms, such as 2,2′-methylene bis(4,6-di-t-butylphenol), 2,2-ethylidene bis(4,6-di-t-butylphenol), and 2,2′-methylene bis(4-methyl-6-t-butylphenol), and the like.

For example, the following combinations provided excellent ageing in polypropylene as shown in the Weatherometer: 0.125 phr of N-methyl-N′-(2,2,6,6-tetramethyl-4-piperidinyl)-2-(3,5-di-t-butyl-4-hydroxyphenyl)-2-methylproprionamide, 0.05 phr of 2,2′,2″-tris[3(3,5-di-t-butyl-4-hydroxyphenyl)propionyloxy]ethyl isocyanurate, and 0.125 phr of 3,9-bis (octadecyloxy)-2,4,8,10-tetraoxa-3,9-diphosphaspiro[5,5]-undecane - more than 2,000 hours.

The compounds are readily incorporated into materials to be patented by dissolving or dispersing them with the materials, in liquids, dispersions, soluations, and solid forms. If the material is a solid, especially a polymeric solid such as rubber or a plastic, the compounds can be admixed using mixers such as Banburys, extruders, two-roll mills, and the like, following conventional techniques. One way to disperse the compounds in plastic materials is to dissolve or suspend the compounds in a solvent or diluent, mix the mixture with a plastic in powder or solution form, and then evaporate the solvent.

Compositions containing the novel combination of compounds can also contain other known compounding ingredients such as fillers like carbon black, silica, metal carbonates, talc, and the like; pigments and colorants; curative ingredients like sulfur and peroxides, and vulcanization accelerators; fungicides; processing aids, reinforcing agents and standard ingredients known to the art. Other ingredients known in the art as ultraviolet light, thermal and/or oxidative stabilizers can also be used.

## Claims

1. N-(substituted cyclic alkyleneimine)-α-(3,5-di-alkyl-4-hydroxyphenyl)-α′,α″-dialkyl acetamides having the general formula

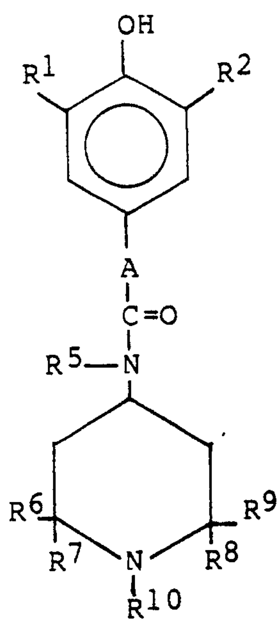

wherein $R^1$ and $R^2$ are hydrogen, alkyl or cycloalkyl groups containing 1 to 12 and 5 to 12 carbon atoms respectively, phenyl, naphthyl or alkaryl derivatives thereof wherein the alkyl groups contain 1 to 8 carbon atoms; $R^5$, $R^6$, $R^7$, $R^8$ and $R^9$ are hydrogen, alkyl or cycloalkyl groups containing 1 to 12 and 5 to 12 carbon atoms respectively, phenyl, naphthyl and alkyl derivatives thereof and alkylcycloalkyl groups wherein the alkyls contain 1 to 8 carbon atoms; A is (1) an alkylidene group of the formula $R^3$-C-$R^4$ wherein $R^3$ and $R^4$ are alkyl groups containing 1 to 18 carbon atoms or (2) cycloalkane groups containing 5 to 12 carbon atoms, phe nyl, naphthyl, alkyl derivatives thereof and alkylcycloalkyl groups, wherein the alkyl

groups contain 1 to 8 carbon atoms; $R^{10}$ is H or an alkyl group containing 1 to 18 carbon atoms, =O or -OH, and acyl group containing 1 to 18 carbon atoms.

2. N-(2,2,6,6-tetraalkyl-4-piperidinyl)-α-(3,5-di-alkyl-4-hydroxyphenyl)-α ,α"-dialkylacetamides Claim 1 wherein $R^1$ contains 1 to 8 carbon atoms; $R^2$ contains 1 to 5 carbon atoms, in (1) $R^3$ and $R^4$ are alkyl containing 1 to 8 carbon atoms; in (2) the cycloalkkane is cyclohexane, methylcyclohexane or cycloheptane; and in $R^5$, $R^6$, $R^7$, $R^8$ and $R^9$ the alkyl groups contain 1 to 8 carbon atoms; and $R^{10}$ is hydrogen or an alkyl group containing 1 to 8 carbon atoms.

3. The acetamides of Claim 2 wherein at least one of $R^1$ and $R^2$ is t-butyl or t-amyl and $R^3$ and $R^4$ are alkyl radicals containing 1 to 4 carbon atoms.

4. N-methyl-N'-(2,2,6,6-tetramethyl-4-piper-dinyl)-2-(3,5-di-t-butyl-4-hydroxyphenyl)-2-methylpropionamide of Claim 3.

5. Bis-{N-(2,2,6,6-tetramethyl-4-piperidinyl)-N'-[2-(3,5-di-t-butyl-4-hydroxyphenyl)-2-methylpropanol]-}-1,6-hexanediamine of Claim 2.

6. N-methyl-N'-(2,2,5,5-tetramethyl-4-piperidinyl)-α-(3,5-di-t-butyl-4-hydroxyphenyl)-cyclohexanecarboxamide of Claim 2.

7. Bis-{N-(2,2,6,6-tetramethyl-4-piperidinyl)-N'-α-[2-(3,5-di-t-butyl-4-hydroxyphenyl)-2-methylbutanoyl]-}-1,6-hexanediamine of Claim 2.

8. Bis-{N-(2,2,6,6-tetramethyl-4-piperidin yl)-N'-α-[2-(3,5-di-t-butyl-4-hydroxyphenyl-2-methylpentanoyl]-}-1,6-hexanediamine of Claim 2.

9. Acetamides of Claim 1 selected from the group consisting of bis-{N-2,2,6,6-tetramethyl-4-piperidinyl)-N'-[2-(3,5-di-t-butyl-4-hydroxyphenyl)-2-methylbutanoyl]}-2-methyl-1,5-pentanediamine; N-butyl-N'-(2,2,5,5-tetramethyl-4-piperidinyl)-2-(3,5-di-t-butyl-4-hydroxyphenyl)-2-methylbutanamide; N-dodecyl-N'-(2,2,5,5-tetramethyl)-4-piperidinyl)-α-(3,5-di-t-butyl-4-hydroxyphenyl)-cyclohexanecarboxamide; N-ethyl-N'-(2,2,6-6-tetramethyl-4-piperidinyl)-2-(3,5-di-t-butyl-4-hydroxyphenyl)-2-methyloctanamide; N-ethyl-N'-(2,2,6,6-tetramethyl-4-piperidinyl)-2-(3,5-di-t-butyl-4-hydroxyphenyl)-2-methylbutanamide; N-ethyl-N'-(1,2,2,6,6-pentamethyl-4-piperidinyl)-2-(3,5-butyl-4-hydroxyphenyl)-2-methylbutanamide; and N-ethyl-N'-(1-acetyl-2,2,6,6-tetramethyl-4-piperidinyl)-2-(3,5-di-t-butyl-4-hydroxyphenyl)-2-methylbutanamide.

10. Compositions comprising (1) organic materials subject to degradation and stabilizing amounts of (2) N-(substituted cyclic alkyleneimine)-α-(3,5-dialkyl-4-hydroxyphenyl)-α',α"-dialkyl acetamides having the formula

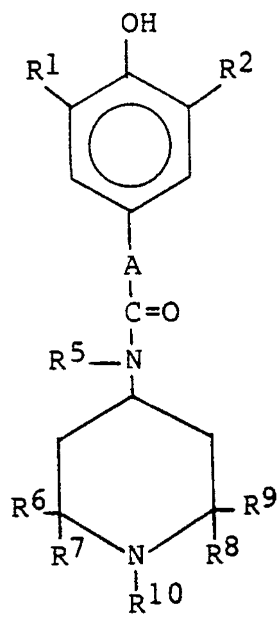

wherein $R^1$ and $R^2$ are hydrogen, alkyl or cycloalkyl groups containing 1 to 12 and 5 to 12 carbon atoms respectively, phenyl, naphthyl or alkaryl derivatives thereof wherein the alkyl groups contain 1 to 8 carbon atoms; $R^5$, $R^6$, $R^7$, $R^8$ and $R^9$ are hydrogen, alkyl or cycloalkyl groups containing 1 to 12 and 5 to 12 carbon atoms respectively, phenyl, naphthyl and alkyl derivatives thereof and alkylcycloalkyl groups wherein the alkyls contain 1 to 8 carbon atoms; A is (1) an alkylidene group of the formula $R^3$- C -$R^4$ wherein $R^3$ and $R^4$ are alkyl groups containing 1 to 18 carbon atoms or (2) cycloalkane groups containing 5

to 12 carbon atoms, phenyl, naphthyl, alkyl derivatives thereof and alkylcycloalkyl groups, wherein the alkyl groups contain 1 to 8 carbon atoms; $R^{10}$ is H or an alkyl group containing 1 to 18 carbon atoms, =O or -OH, and acyl group containing 1 to 18 carbon atoms.

11. Compositions of Claim 10 wherein $R^1$ contains 1 to 8 carbon atoms; $R^2$ contains 1 to 5 carbon atoms; $R^3$ and $R^4$ contain 1 to 8 carbon atoms; (2) is cyclohexyl, methylcyclohexyl, cycloheptyl, in $R^5$, $R^6$, $R^7$ and $R^8$ the alkyl groups contain 1 to 8 carbon atoms; and $R^{10}$ is hydrogen or an alkyl group containing 1 to 8 carbon atoms.

12. Compositions of Claim 11 wherein at least one of $R^1$ and $R^2$ is t-butyl or t-amyl and $R^3$ and $R^4$ are alkyl radicals containing 1 to 4 carbon atoms.

13. Compositions of Claim 12 wherein in (1) said organic material is a polymer and (2) is selected from the group consisting of N-methyl-N'-(2,2,6,6-tetramethyl-4-piperidinyl)-2-(3,5-di-t-butyl-4-hydroxyphenyl)-2-methylpropionamide; bis-{N-(2,2,6,6-tetramethyl-4-piperidinyl)-N'-[2-(3,5-di-t-butyl-4-hydroxyphenyl)-2 -methylpropanol]}-1,6-hexanediamine; N-methyl-N'-(2,2,5,5-tetramethyl-4-piperidinyl)-α-(3,5-di-t-butyl-4-hydroxyphenyl)-cyclohexanecarboxamide; bis-{N-(2,2,6,6-tetramethyl-4-piperidinyl)N'-[2-(3,5-di-t-butyl-4-hydroxyphenyl)-2-methylbutanoyl]}-1,6-hexanediamine; and bis-{N-(2,2,6,6-tetramethyl-4-piperidinyl)-N'-[2-(3,5-di-t-butyl-4-hydroxyphenyl)-2-methylpentanoyl]}-1,6-hexanediamine.

14. Compositions of Claim 10 wherein in (1) said organic material is a polymer and (2) is selected from the group consisting of bis-{N-2,2,6,6-tetramethyl-4-piperidinyl)-N'-[2-(3,5-di-t-butyl-4-hydroxyphenyl)-2-methylbutanoyl]}-2-methyl-1,5-pentanediamine; N-butyl-N'-(2,2,5,5-tetramethyl-4-piperidinyl)-2-(3,5-di-t-butyl-4-hydroxyphenyl)-2-methylbutanamide; N-dodecyl-N'-(2,2,5,5-tetramethyl)-4-piperidinyl)-α-(3,5-di-t-butyl-4-hydroxyphenyl)-cyclohexanecarboxamide; N-ethyl-N'-(2,2,6,6-tetramethyl-4-piperidinyl)-2-(3,5-di-t-butyl-4-hydroxyphenyl)-2-methyloctanamide; N-ethyl-N'-(2-2,6,6-tetramethyl-4-piperidinyl)-2-(3,5-di-t-butyl-4-hydroxyphenyl)-2-methylbutanamide; N-ethyl-N'-(1,2,2,6-6-pentamethyl-4-piperidinyl)-2-(3,5-butyl-4-hydroxyphenyl)-2-methylbutanamide; and N-ethyl-N'-(1-acetyl-2,2-6,6-tetramethyl-4-piperidinyl)-2-(3,5-di-t-butyl-4-hydroxyphenyl)-2-methylbutanamide.

15. A process for preparing N-(substituted cyclic alkyleneimine)-α-(3,5-di-alkyl-4-hydroxyphenyl)-α,α'-dialkyl acetamides having the formula

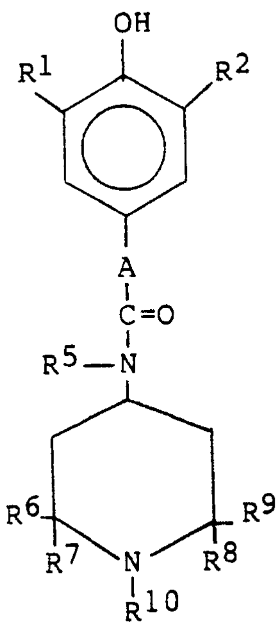

wherein $R^1$ and $R^2$ are hydrogen, alkyl or cycloalkyl groups containing 1 to 12 and 5 to 12 carbon atoms respectively, phenyl, naphthyl or alkaryl derivatives thereof wherein the alkyl groups contain 1 to 8 carbon atoms; $R^5$, $R^6$, $R^7$, $R^8$ and $R^9$ are hydrogen, alkyl or cycloalkyl groups containing 1 to 12 and 5 to 12 carbon atoms respectively, phenyl, naphthyl and alkyl derivatives thereof and alkylcycloalkyl groups wherein the alkyls contain 1 to 8 carbon atoms; A is (1) an alkylidene group of the formula $R^3$-C-$R^4$ wherein $R^3$ and $R^4$ are alkyl groups containing 1 to 18 carbon atoms or (2) cycloalkane groups containing 5 to 12 carbon atoms, phenyl, naphthyl, alkyl derivatives thereof and alkylcycloalkyl groups, wherein the alkyl groups contain 1 to 8 carbon atoms; $R^{10}$ is H or an alkyl group containing 1 to 18 carbon atoms, =O or -OH, and acyl group containing 1 to 18 carbon atoms, comprising reacting together a 2,6-dialkylphenol

having the formula

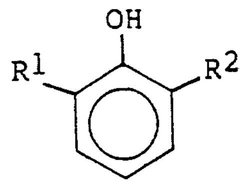

wherein $R^1$ and $R^2$ have the meanings above, a ketone selected from the group consisting of dialkyl ketones, cycloalakanones, alkcycloalkanones, and alkaryl ketones wherein the alkyl groups contain 1 to 18 carbon atoms, a haloform selected from the group consisting of chloroform and bromoform, an alkali metal hydroxide and piperidine having the general formula

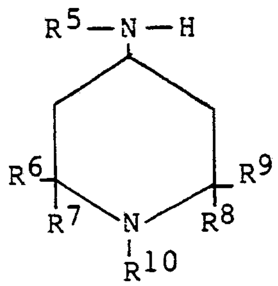

wherein $R^5$, $R^6$, $R^7$, $R^8$, $R^9$ and $R^{10}$ have the meanings above.

16. A process of Claim 13 wherein $R^1$ contains 1 to 8 carbon atoms and $R^2$ contains 1 to 5 carbon atoms, the ketone is a dialkyl ketone of the formula

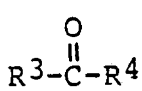

wherein $R^3$ and $R^4$ are alkyl radicals containing 1 to 8 carbon atoms, cyclopentanone, cyclohexanone, cycloheptanone and alkyl derivatives thereof wherein the alkyl groups contain 1 to 4 carbon atoms, in $R^5$, $R^6$, $R^7$, $R^8$ and $R^9$ the alkyl groups contain 1 to 8 carbon atoms.

European Patent Office

# EUROPEAN SEARCH REPORT

Application number
EP 88103130.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A2 - 0 172 413 (SUMITOMO)<br>* Claims 1,15 *<br>-- | 1,10,<br>15,16 | C 07 D 211/58<br>C 08 K 5/34 |
| A | EP - A1 - 0 062 322 (GOODRICH)<br>* Claims 1,4 *<br>-- | 1,15 | |
| A | US - A - 4 526 972 (SPERANZA)<br>* Abstract *<br>---- | 1,10 | |
| | | | TECHNICAL FIELDS SEARCHED (Int. Cl.4) |
| | | | C 07 D 211/00 |

The present search report has been drawn up for all claims

| Place of search | Date of completion of the search | Examiner |
|---|---|---|
| VIENNA | 31-05-1988 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document